# EUROPÄISCHE PATENTSCHRIFT

(11) **EP 0 789 550 B1**
(45) Veröffentlichungstag und Bekanntmachung des Hinweises auf die Patenterteilung: **20.12.2000**
(21) Anmeldenummer: 95937830.8
(22) Anmeldetag: 24.10.1995
(51) Int. Cl.: A61K 7/06, A61K 7/00

(54) **HAARFESTLEGEMITTEL**
HAIR-SETTING AGENTS
PRODUITS FIXATEURS POUR LES CHEVEUX

(30) Priorität: 02.11.1994 DE 4438849
(43) Veröffentlichungstag der Anmeldung: 20.08.1997
(73) Patentinhaber: Henkel Kommanditgesellschaft auf Aktien, 40191 Düsseldorf (DE)
(72) Erfinder: EMMERLING, Winfried, D-41469 Neuss (DE); NIEMANN, Annedore, D-40721 Hilden (DE); SCHIEFERSTEIN, Ludwig, D-40882 Ratingen (DE)
(86) Internationale Anmeldenummer: EP9504161
(87) Internationale Veröffentlichungsnummer: WO9614050

(56) Entgegenhaltungen:
- EP-A- 0 418 676
- EP-A- 0 455 081
- DE-A- 4 241 118
- CHEMICAL ABSTRACTS, vol. 96, no. 22, 31.Mai 1982 Columbus, Ohio, US; abstract no. 187093e, Seite 401; & JP,A,82 011 906 (SEITETSU KAGAKU CO.,LTD) 21.Januar 1982

## Beschreibung

Die Erfindung betrifft Haarfestlegemittel mit Polyurethan-Polymeren.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung bestimmter festigender Wirkstoffe aufbauen bzw. für einen längeren Zeitraum aufrechterhalten lassen.

Diese festigenden Wirkstoffe, bei denen es sich in der Regel um polymere Verbindungen handelt, können in übliche Haarreinigungs- oder -konditioniermittel eingearbeitet werden. In vielen Fällen ist es aber vorteilhaft, sie in Form spezieller Haarfestlegemittel wie Haarfestiger oder Haarsprays anzuwenden.

Es gibt nun in jüngster Zeit eine Reihe von Entwicklungen auf dem Haarkosmetikgebiet, die einen Bedarf an neuartigen festigenden Wirkstoffen bzw. neuen Formulierungsformen geweckt haben. Viele dieser Entwicklungen beruhen dabei nicht auf anwendungstechnischen Nachteilen oder Unzulänglichkeiten der bekannten Mittel, sondern z.B. auf Umweltschutz-Gesichtspunkten, gesetzlichen Auflagen oder anderen "nicht-technischen" Ursachen.

Es besteht dann die Aufgabe, entsprechende Mittel zu entwickeln, die hinsichtlich der anwendungstechnischen Eigenschaften, beispielsweise dem Sprühverhalten und der Trocknungszeit bei Haarsprays, die vom Verbraucher gesteckten Erwartungen erfüllen.

So wird insbesondere verstärkt ein Übergang von Mitteln auf Basis flüchtiger Alkohole zu Mitteln auf wäßriger Basis angestrebt. Dabei ist ein Hauptproblem die geringere Flüchtigkeit von Wasser im Vergleich zu den Alkoholen, was sich in längeren Trocknungszeiten auf dem Haar niederschlägt. Weiterhin ist aufgrund der häufig schlechteren Löslichkeit polymerer Verbindungen in wäßrigen Systemen diese Umstellung häufig mit dem Nachteil verbunden, daß beim Aufbringen der gewünschten Polymermenge auf das Haar Wasser zwangsläufig in solchen Mengen auf das Haar gelangt, daß die Trocknungszeiten unakzeptabel lang werden.

Dieser Nachteil tritt dagegen nicht auf, wenn diese nur unzureichend wasserlöslichen Polymeren gleichmäßig und stabil in dem Mittel dispergiert werden können. In diesem Fall gelangt bei der Anwendung mit der gewünschten Polymermenge nur eine weit niedrigere Wassermenge auf das Haar.

Aus der DE-A1-424118 sind Haarsprays und Haarfestiger mit kationischen Polyurethanen und Polyharnstoffen auf ethanolischer und wäßrig-ethanolischer Basis bekannt.

Besondere Aufmerksamkeit haben in jüngster Zeit Mittel auf Basis Wasser/Dimethylether gefunden wie sie beispielsweise in der EP-A1-418676 beschrieben werden.

Dabei ist zu beachten, daß dieses binäre System eine Mischungslücke aufweist. wenn der Gehalt an Dimethylether zwischen ca. 34% und ca. 94% liegt. Das Auftreten dieser Mischungslücke kann zwar durch Zugabe anderer Komponenten vermieden werden, z. B. wenn das System mehr als ca. 12 Gew.-% an Ethanol enthält, doch ist es häufig gerade der Gehalt dieser Komponenten, der vermieden oder begrenzt werden soll.

Es ware daher für eine Reihe von Anwendungen vorteilhaft, wenn das Auftreten der Mischungslücke durch solche Zusatzstoffe vermieden werden könnte, die sonst keine unerwünschten Eigenschaften aufweisen oder ohnehin als Wirkstoffkomponente in dem Mittel enthalten sind.

Es wurde nun überraschenderweise gefunden, daß als Filmbildner in Haarfestlegemitteln geeignete Polyurethane den Existenzbereich der Mischungslücke in Wasser/Dimethylether-Systemen in dramatischer Weise beeinflussen.

Gegenstand der Erfindung sind daher Haarfestlegemittel, enthaltend
- 10 - 60 Gew.-% Dimethylether,
- 39,9- 89,9 Gew.-% Wasser,
- 0,1- 15,0 Gew.-% Polyurethan und
- 0,0- 5,0 Gew.-% Alkanol mit 1-4 Kohlenstoffatomen.

Die Polyurethane bestehen aus mindestens zwei verschiedenen Monomertypen,
- einer Verbindung (A) mit mindestens 2 aktiven Wasserstoffatomen pro Molekül und
- einem Di- oder Polyisocyanat (B).

Bei den Verbindungen (A) kann es sich beispielsweise um Diole, Triole, Diamine, Triamine, Polyetherole und Polyesterole handeln. Dabei werden die Verbindungen mit mehr als 2 aktiven Wasserstoffatomen üblicherweise nur in geringen Mengen in Kombination mit einem großen Überschuß an Verbindungen mit 2 aktiven Wasserstoffatomen eingesetzt.

Beispiele für Verbindungen (A) sind Ethylenglykol, 1,2- und 1,3-Propylenglykol, Butylenglykole, Di-, Tri-, Tetra- und Poly-Ethylen- und -Propylenglykole, Copolymere von niederen Alkylenoxiden wie Ethylenoxid, Propylenoxid und Butylenoxid, Ethylendiamin, Propylendiamin, 1,4-Diaminobutan, Hexamethylendiamin und α,w-Diamine auf Basis von langkettigen Alkanen oder Polyalkylenoxiden.

Polyurethane, bei denen die Verbindungen (A) Diole, Triole und Polyetherole sind, können erfindungsgemäß bevorzugt sein. Insbesondere Polyethylenglykole und Polypropylenglykole mit Molmassen zwischen 200 und 3000, insbesondere zwischen 1600 und 2500, haben sich in einzelnen Fällen als besonders geeignet erwiesen.

Polyesterole werden üblicherweise durch Modifizierung der Verbindung (A) mit Dicarbonsäuren wie Phthalsäure, Isophthalsäure und Adipinsäure erhalten.

Als Verbindungen (B) werden überwiegend Hexamethylendiisocyanat, 2,4- und 2,6-Toluoldiisocyanat, 4,4'-Methylendi(phenylisocyanat) und insbesondere Isophorondiisocyanat eingesetzt.

Weiterhin können die erfindungsgemäß verwendeten Polyurethane noch Bausteine wie beispielsweise Diamine als Kettenverlängerer und Hydroxycarbonsäuren enthalten. Dialkylolcarbonsäuren wie beispielsweise Dimethylolpropionsäure sind besonders geeignete Hydroxycarbonsäuren. Hinsichtlich der weiteren Bausteine besteht keine grundsätzliche Beschränkung dahingehend, ob es sich um nichtionische, anionischen oder kationische Bausteine handelt.

Bezüglich weiterer Informationen über den Aufbau und die Herstellung der Polyurethane wird ausdrücklich auf die Artikel in den einschlägigen Übersichtswerken wie Römpps Chemie-Lexikon und Ullmanns Enzyklopädie der technischen Chemie Bezug genommen.

Als in vielen Fälle erfindungsgemäß besonders geeignet haben sich Polyurethane erwiesen, die wie folgt charakterisiert werden können:
- ausschließlich aliphatische Gruppen im Molekül
- keine freien Isocyanatgruppen im Molekül
- Polyether- und Polyesterpolyurethane
- anionische Gruppen im Molekül.

Es hat sich ebenfalls in einigen Fällen als vorteilhaft erwiesen, wenn das Polyurethan in dem System nicht gelöst, sondern stabil dispergiert ist.

Weiterhin hat es sich als für die Herstellung der erfindungsgemäßen Mittel als vorteilhaft erwiesen, wenn die Polyurethane nicht direkt mit den weiteren Komponenten gemischt, sondern in Form von wäßrigen Dispersionen eingebracht wurden. Solche Dispersionen weisen üblicherweise einen Feststoffgehalt von ca. 20-50 %, insbesondere etwa 35-45% auf und sind auch kommerziell erhältlich.

Die erfindungsgemäßen Haarbehandlungsmittel enthalten das Polyurethan in in Mengen von 0,1 bis 15 Gew.-%, insbesondere 0,5 bis 10 Gew.-%, bezogen auf das gesamte Mittel.

In einer bevorzugten Ausführungsform kann es vorteilhaft sein, die erfindungsgemäßen Mittel hinsichtlich der Komponenten Wasser und Dimethylether so zu formulieren, daß das Mischungsverhältnis in der Mischungslücke liegt, aufgrund der Anwesenheit des Polyurethan jedoch keine Phasentrennung in eine wäßrige und eine Dimethylether-Phase auftritt. Mischungen, die, bezogen auf das binäre System, 34 - 60 % Dimethylether und 40 - 66 % Wasser enthalten, können besonders bevorzugt sein. Mischungen mit 40 - 50 % Dimethylether können ganz besonders bevorzugt sein.

In einer weiteren bevorzugten Ausführungsform kann es vorteilhaft sein, die erfindungsgemäßen Mittel hinsichtlich der Komponenten Wasser und Dimethylether so zu formulieren, daß das Mischungsverhältnis in der Mischungslücke liegt, und trotz Anwesenheit des Polyurethans eine Phasentrennung in eine wäßrige und eine Dimethylether-Phase auftritt.

In einigen Fällen hat es sich jedoch als vorteilhaft erwiesen, das Wasser/Dimethylether-Verhältnis so zu wählen, daß es außerhalb der binären Mischungslücke liegt.

Die erfindungsgemäßen Mittel können bis zu 5 Gew.-% an Alkanolen mit 1-4 Kohlenstoffatomen, inbsbesondere Ethanol und Isopropanol enthalten. In einer bevorzugten Ausführungsform werden die erfindungsgemäßen Mittel aber ganz ohne diese Alkanole formuliert.

Weiterhin können die erfindungsgemäßen Mittel in einer bevorzugten Ausführungsform ein wasserlösliches Polymer aus der Gruppe der nichtionischen, anionischen, amphoteren und zwitterionischen Polymeren enthalten.

Als wasserlösliche Polymere sind dabei solche Polymeren zu verstehen, die bei Raumtemperatur in Wasser zu mehr als 2,5 Gew.-% löslich sind.

Erfindungsgemäß bevorzugte wasserlösliche Polymere sind nichtionisch. Geeignete nichtionogene Polymere sind beispielsweise:
- Polyvinylpyrrolidone, wie sie beispielsweise unter der Bezeichnung Luviskol^{R} (BASF) vertrieben werden. Polyvinylpyrrolidone sind bevorzugte nichtionische Polymere im Rahmen der Erfindung.
- Vinylpyrrolidon/Vinylester-Copolymere, wie sie beispielsweise unter dem Warenzeichen Luviskol^{R} (BASF) vertrieben werden. Luviskol^{R} VA 64 und Luviskol^{R} VA 73, jeweils Vinylpyrrolidon/Vinylacetat-Copolymere, sind besonders bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{R} und Benecel^{R} (AQUALON) vertrieben werden.

Geeignete amphotere Polymere sind beispielsweise die unter den Bezeichnungen Amphomer^{R} und Amphomer^{R} LV-71 (DELFT NATIONAL) erhältlichen Octylacrylamid/Methylmethacrylat/tert. Butylaminoethylmethacrylat/2-Hydroxypropylmethacrylat-Copolymere.

Geeignete zwitterionische Polymere sind beispielsweise die in den deutschen Patentanmeldungen DE 39 29 973, DE 21 50 557, DE 28 17 369 und DE 37 08 451 offenbarten Polymerisate. Acrylamidopropyltrimethylammoniumchlorid/Acrylsäure- bzw. -Methacrylsäure-Copolymerisate und deren Alkali-und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette^{R} (AMERCHOL) im Handel erhältlich sind.

Erfindungsgemäß geeignete anionische Polymere sind u. a.:
- Vinylacetat/Crotonsäure-Copolymere, wie sie beispielsweise unter den Bezeichnungen Resyn^{R} (NATIONAL STARCH), Luviset^{R} (BASF) und Gafset^{R} (GAF) im Handel sind.
- Vinylpyrrolidon/Vinylacrylat-Copolymere, erhältlich beispielsweise unter dem Warenzeichen Luviflex^{R} (BASF). Ein bevorzugtes Polymer ist das unter der Bezeichnung Luviflex^{R} VBM-35 (BASF) erhältliche Vinylpyrrolidon/Acrylat-Terpolymere.
- Acrylsäure/Ethylacrylat/N-tert.Butylacrylamid-Terpolymere, die beispielsweise unter der Bezeichnung Ultrahold^{R} strong (BASF) vertrieben werden.

In den Fällen, in denen das Polyurethan ionische Gruppen enthält, hat es sich als zweckmäßig erwiesen, wenn das wasserlösliche Polymere nichtionogen oder von gleicher Ionogenität ist.

Die erfindungsgemäßen Haarbehandlungsmittel enthalten das wassserlösliche Polymer in Abhängigkeit vom Typ des Haarbehandlungsmittels, der keinen Einschränkungen unterliegt, bevorzugt in Mengen von 0,01 bis 20 Gew.-%, insbesondere 0,1 bis 10 Gew.-%, bezogen auf das gesamte Mittel.

Die Polyurethane und die wasserlöslichen Polymere sind bevorzugt in einem Mengenverhältnis von 1:10 bis 10:1 in den erfindungsgemäßen Mitteln enthalten. Ein Mengenverhältnis von 2:1 bis 1:1 hat sich in vielen Fällen als besonders geeignet erwiesen.

Bei den erfindungsgemäßen Haarfestlegemitteln handelt es sich insbesondere um Haarfestiger, Haarsprays und Fönwellen. Haarsprays sind eine besonders bevorzugte Ausführungsform der erfindungsgemäßen Haarfestlegemittel.

Die erfindungsgemäßen Mittel können weiterhin in einer ebenfalls bevorzugten Ausführungsform mit Hilfe eines Treibmittels auch als Schaumaerosol formuliert sein.

Weitere Bestandteile der erfindungsgemäßen Mittel können beispielsweise sein:
- anionische Tenside, wie beispielsweise Fettalkylsulfate und -ethersulfate,
- kationische Tenside, wie beispielsweise quartäre Ammoniumverbindungen,
- zwitterionische Tenside, wie beispielsweise Betaine,
- ampholytische Tenside,
- nichtionogene Tenside, wie beispielsweise Alkylpolyglycoside und ethoxylierte Fettalkohole,
- Strukturanten wie Glucose und Maleinsäure,
- haarkonditionierende Verbindungen wie Phospholipide, beispielsweise Sojalecithin, Ei-Lecithin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler, wie Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes,
- Wirkstoffe wie Panthenol, Allantoin, Pyrrolidoncarbonsäuren und deren Salze, Pflanzenextrakte und Vitamine,
- Lichtschutzmittel,
- Konsistenzgeber wie Zuckerester, Polyolester oder Polyolalkylether,
- Fette und Wachse, wie Walrat, Bienenwachs, Montanwachs, Paraffine und Fettalkohole,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Treibmittel wie Propan-Butan-Gemische, N₂0, Dimethylether, C0₂ und Luft sowie
- Antioxidantien,

Gegenstand der Erfindung ist auch die Verwendung der erfindungsgemäßen Mittel zur Behandlung von Keratinfasern, insbesondere von Haaren.

Das folgende Beispiel soll die Erfindung näher erläutern.

### Beispiel

Alle Angaben sind Gewichtsteile.

### 1. Aerosol-Haarspray

| | |
|---|---|
| Alberdingk U^{R}500¹ | 5,0 |
| Luviskol^{R}VA64² | 3,0 |
| Panthenol | 0,5 |
| Dimethylether | 40,0 |
| Wasser | ad 100 |

| | |
|---|---|
| ¹ anionische Polyether-Polyurethan-Dispersion (40% in Wasser) (ALBERDINGK BOLEY) | |
| ² Vinylacetat-Vinylpyrrolidon-Copolymer (BASF) | |

## Patentansprüche

1. Haarfestlegemittel, enthaltend
- 10 - 60 Gew.-% Dimethylether,
- 39,9- 89,9 Gew.-% Wasser,
- 0,1- 15,0 Gew.-% Polyurethan bestehend aus mindestens zwei verschiedenen Monomertypen,
- einer Verbindung (A) mit mindestens 2 aktiven Wasserstoffatomen pro Molekül und
- einem Di- oder Polyisocyanat (B)
- 0.0- 5.0 Gew.-% Alkanol mit 1-4 Kohlenstoffatomen.

2. Mittel nach Anspruch 1, dadurch gekennzeichnet, daß das Polyurethan anionische Gruppen enthält.

3. Mittel nach Anspruch 1 oder 2, dadurch gekennzeichnet, daß das Polyurethan in dem Mittel dispergiert ist.

4. Mittel nach einem der Ansprüche 1 bis 3, dadurch gekennzeichnet, daß es weiterhin ein wasserlösliches Polymer enthält.

5. Mittel nach Anspruch 4, dadurch gekennzeichnet, daß das wasserlösliche Polymer nichtionisch ist.

6. Mittel nach einem der Ansprüche 4 oder 5, dadurch gekennzeichnet, daß das wasserlösliche Polymer Polyvinylpyrrolidon oder ein Copolymer, gebildet aus Vinylpyrrolidon- und Vinylester-Monomeren, ist.

7. Mittel nach einem der Ansprüche 4 bis 6, dadurch gekennzeichnet, daß das wasserlösliche Polymer in einer Menge von 0,01 bis 20 Gew.-%, insbesondere von 0,1 bis 10 Gew.-%, enthalten ist.

8. Verwendung eines Mittels nach einem der Ansprüche 1 bis 7 zur Behandlung von Haaren.

## Claims

1. Hair setting preparation containing
- 10 to 60% by weight dimethyl ether,
- 39.9 to 89.9% by weight water,
- 0.1 to 15.0% by weight polyurethane consisting of at least two different monomer types, namely: a compound (A) containing at least two active hydrogen atoms per molecule and a di- or polyisocyanate (B), and
- 0.0 to 5.0% by weight C₁₋₄ alkanol.

2. A preparation as claimed in claim 1, characterized in that the polyurethane contains anionic groups.

3. A preparation as claimed in claim 1 or 2, characterized in that the polyurethane is dispersed in the preparation.

4. A preparation as claimed in any of claims 1 to 3, characterized in that it additionally contains a water-soluble polymer.

5. A preparation as claimed in claim 4, characterized in that the water-soluble polymer is nonionic.

6. A preparation as claimed in claim 4 or 5, characterized in that the water-soluble polymer is polyvinyl pyrrolidone or a copolymer formed from vinyl pyrrolidone and vinyl ester monomers.

7. A preparation as claimed in any of claims 4 to 6, characterized in that the water-soluble polymer is present in a quantity of 0.01 to 20% by weight and more particularly in a quantity of 0.1 to 10% by weight.

8. The use of the preparation claimed in any of claims 1 to 7 for the treatment of hair.

## Revendications

1. Agent de fixation pour cheveux contenant
• de 10 à 60 % en poids d'éther diméthylique,
• de 39,9 à 89,9 % en poids d'eau,
• de 0,1 à 15,0 % en poids de polyuréthane constitué d'au moins deux types de monomères différents,
un composé (A) avec au moins deux atomes d'hydrogène actif par molécule, et
un di- ou polyisocyanate (B),
• de 0,0 à 5,0 % en poids d'alcanol comportant de 1 à 4 atomes de carbone.

2. Agent selon la revendication 1,
caractérisé en ce que
le polyuréthane contient des groupes anioniques.

3. Agent selon les revendications 1 ou 2,
caractérisé en ce que
le polyuréthane est dispersé dans l'agent.

4. Agent selon l'une quelconque des revendications 1 à 3,
caractérisé en ce qu'
il contient en outre un polymère soluble dans l'eau.

5. Agent selon la revendication 4,
caractérisé en ce que
le polymère soluble dans l'eau est non ionique.

6. Agent selon l'une quelconque des revendications 4 ou 5,
caractérisé en ce que
le polymère soluble dans l'eau est la polyvinylpyrrolidone ou un copolymère formé de vinylpyrrolidone et de monomères d'ester vinylique.

7. Agent selon l'une quelconque des revendications 4 à 6,
caractérisé en ce que
le polymère soluble dans l'eau est contenu en une quantité de 0,01 à 20 % en poids, en particulier de 0,1 à 10 % en poids.

8. Utilisation d'un agent selon l'une quelconque des revendications 1 à 7 pour le traitement des cheveux.
